# EUROPEAN PATENT APPLICATION

(11) **EP 3 508 271 A1**
(43) Date of publication of application: **10.07.2019**
(21) Application number: 17845100.1
(22) Date of filing: 25.07.2017
(51) Int. Cl.: B01J 23/745, B01J 35/10, C10G 2/00, B01J 37/10

(54) **MONODISPERSED IRON-BASED CATALYST FOR FISCHER-TROPSCH PROCESS, PREPARATION METHOD THEREFOR AND USE THEREOF**

(30) Priority: 31.08.2016 CN 201610785839
(71) Applicant: Wuhan Kaidi Engineering Technology Research Institute Co., Ltd., Wuhan, Hubei 430212 (CN)
(72) Inventor: GUO, Li, Wuhan Hubei 430212 (CN); CHEN, Jiangang, Wuhan Hubei 430212 (CN); ZHANG, Yanfeng, Wuhan Hubei 430212 (CN); JIN, Jiaqi, Wuhan Hubei 430212 (CN); SHI, Youliang, Wuhan Hubei 430212 (CN); ZHENG, Shenke, Wuhan Hubei 430212 (CN); GONG, Yan, Wuhan Hubei 430212 (CN)
(74) Representative: Zeuner Summerer Stütz
(86) International application number: PCT/CN2017/094223
(87) International publication number: WO 2018/040798

(57) **Abstract**

Disclose are a monodispersed iron-based catalyst for the Fischer-Tropsch process, a preparation method therefor and the use thereof. The catalyst comprises a carrier, i.e. silicon dioxide and an active component, i.e. iron. The carrier, i.e. silicon dioxide has a mesoporous spherical particle structure, and the carrier, i.e. silicon dioxide covers the active component, i.e. iron. The active component, i.e. iron is of a nanoparticle shape and is distributed uniformly. The particle size of the carrier, i.e. silicon dioxide particle is 140 - 160 nm. In the preparation method therefor, the pressure of CO2 is adjusted, so that the morphology of the catalyst is changed from a disc-shaped stack into a uniform spherical shape. The iron nanoparticles are thus covered in the interior of the spherical silicon dioxide, sintering of the iron nanoparticles is prevented, and the appearance of an ingredient which is difficult to reduce is also avoided. The iron-based catalyst can be applied to a process route for preparing an α-olefin, wherein the α-olefin is directly synthesized by using a synthetic gas as a raw material under the action of the iron-based catalyst. The iron-based catalyst has a high selectivity for a long chain α-olefin.

## Description

### FIELD OF THE INVENTION

This disclosure relates to the field of Fischer-Tropsch synthesis, and more particularly to a monodisperse iron-based catalyst for Fischer-Tropsch synthesis, preparation method and use thereof.

### BACKGROUND

Long chain alpha-olefins are monoolefins or mixed olefins whose unsaturated bonds are located at the end of chain hydrocarbons having 4-16 carbons. Due to the active sites (alkene bonds) in the molecules, alpha-olefin is easy to access to various functional groups to convert into alcohols, acids and esters and other compounds, so it is widely used in the production of fine chemicals such as surfactants and plasticizers.

At present, alpha-olefins are mainly used as comonomers in the production of polyethylene, accounting for 44.1% of the total consumption. The most widely used copolymers are C₄ (1-butene), C₆ (1-hexene) and C₈ (1-octene). The use of these copolymers in the production of high density polyethylene and linear low density polyethylene (HDPE/LLDPE) can improve the density, tear resistance and tensile strength of polyethylene (PE). Second, alpha olefins are also used in the production of high carbon alcohols. The final products are plasticizers and detergents, which account for 19.6% of the total consumption. High carbon alcohols are the basic raw materials for producing surfactants and plasticizers, and the consumption in the world about ten million tons per year. Octanol is mainly used for the preparation of various esters, and the sales amount thereof are about one billion dollars annually in the world. Heptanol can be used to prepare spices and flavors with high value. Alpha-olefins are also used in the production of poly-alpha-olefins (PAO), accounting for 16.6% of the total consumption. PAO is the raw material of the base oil for high-end lubricating oil, with a high viscosity index and a price 2-3 times higher than that of mineral oils. In addition, alpha-olefins are also used as chemical raw materials of emulsifiers, leather treatment agents, lubricant additives, rust inhibitors, fabric finishing agents and paper. At present, the total production capacity of alpha-olefins abroad is about 2.12 million tons, and the domestic production capacity is about 260,000 tons. Moreover, China lacks the capacity to produce long-chain alpha-olefins, and 1-octene and 1-hexene are almost entirely dependent on imports.

Conventional production processes of alpha-olefins include paraffin pyrolysis or ethylene oligomerization. Paraffin pyrolysis generally uses deoiled refined wax as raw material. The wax is preheated and mixed with steam, and allowed to react in a tubular pyrolysis furnace at 550°C. Ethylene oligomerization uses triethylaluminium as a catalyst to produced alpha-olefins. Specifically, ethylene monomers are polymerized to form a certain chain length of olefins through controlled chain growth reaction, which can be divided into two-step method, one-step method and SHOP process, etc. The traditional production process of alpha-olefins is based on wax or ethylene. The raw material is petroleum. With the shrinkage of oil and gas resources worldwide, this production process needs to be transformed urgently. Therefore, the diversification of energy raw materials is being carried out at home and abroad, and the non-petroleum route of indirect conversion of coal, biomass or natural gas into clean liquid fuels and chemicals through syngas has been paid more and more attention.

In recent years, according to the difference of core technology, olefin preparation technology can be roughly divided into the following types. The first method is to prepare light olefins from methanol or dimethyl ether as taught in Chinese Patent Application NO. CN101265151A. The raw materials of methanol or dimethyl ether are fed into the reaction zone of the reactor through a feed distributor and contact with molecular sieve catalysts to form a mixture of light olefins, dienes, oxides and carbon tetraolefins. Second, to prepare an iron/activated carbon catalyst used for the synthesis of ethylene, propylene and butene from syngas, as shown in Chinese Patent Application Nos. CN101265149A and CN1537674A. The catalyst is characterized by its specific chemical composition. Third, as shown in Chinese Patent Application Nos. CN1444551A, CN1545520A, CN1443149A and CN1440311A, the oligomerization production of straight-chain alpha-olefins from ethylene for ligand and catalyst systems and processes is disclosed. Fourth, as shown in Chinese Patent Application Nos. CN1403425A and CN1421424A, the methods and equipment for preparing alpha-olefins from wax cracking or high-carbon alpha-olefins from high-carbon alkyl aluminum is disclosed. Fifth, as shown in Chinese Patent Application Nos. CN1284898A and CN1515359A, a method for producing self-supporting precipitating iron catalyst particles for Fischer-Tropsch slurry bed process is disclosed.

The aforesaid alpha-olefin preparation technologies either rely on dwindling petroleum materials, or through Fischer-Tropsch synthesis. Fischer-Tropsch synthesis is a technology that converts coal, natural gas, petroleum coke and other carbon-containing raw materials into high-quality clean syngas (CO/H₂). Fischer-Tropsch reaction is a complex process. The products include alkanes, olefins, aldehydes, alcohols and organic acids. Iron-based catalysts are often used in the preparation of alpha-olefins by Fischer-Tropsch synthesis. However, the existing iron-based catalysts tend to aggregate and grow up in the reaction process, resulting in the decrease of catalyst activity, and the stability of catalysts and the selectivity of long-chain alpha-olefins are not high.

### SUMMARY

One objective of the disclosure is to provide a monodisperse iron-based catalyst for Fischer-Tropsch synthesis and its preparation method and application. The iron-based catalyst exhibits excellent performance and high selectivity for long chain alpha-olefins.

Disclosed is a monodisperse iron-based catalyst for Fischer-Tropsch synthesis, the catalyst comprising silica and iron; the silica is in the form of a mesoporous spherical particle; the iron is in the form of nanoparticles evenly distributed and encapsulated in the silica; a particle size of the silica is between 140 and 160 nm, and a mesopore of the silica is between 2 and 9 nm.

The iron can account for 5-40 wt. % of the catalyst, and the balance is the silica.

The particle size of the silica can be between 150 and 160 nm, and the mesopore of the silica can be between 2.1 and 5.7 nm.

The particle size of the silica can be between 150 and 155 nm, and the mesopore of the silica can be between 3.3 and 4.1 nm.

Also provided is a method of preparing the aforesaid catalyst, the method comprising:
1) mixing ethanol and water with a volume ratio of 1-10: 1-10 to yield an ethanol water, adding 0.005-0.02 g/mL of an organic amine to the ethanol water, to yield a mixture;
2) adding iron nanoparticles to the mixture obtained in 1), followed by addition of 0.05-0.2 g/mL of tetraethyl orthosilicate, to yield a product;
3) introducing 1-15 megapascal CO₂ to the product obtained in 2), heating to 35-45°C for reaction, cooling, and releasing the CO₂, to yield a solid product; and
4) washing the solid product obtained in 3) with water, drying, calcining, to yield the catalyst.

3) can be performed in a reactor filled with 6-9 megapascal of CO₂ at a temperature of 40-45°C for 22-26 hours.

In 4), the calcining temperature can be 500-560°C, and the calcining time can be 4.5-5.5 hours.

In 2), the iron nanoparticles can account for 5-40 wt. % of the mixture.

A method of preparing alpha-olefin through Fischer-Tropsch synthesis, the method comprising employing syngas as a material and using the catalyst. The reaction conditions of the Fischer-Tropsch synthesis are as follows: reaction temperature 190-360°C, reaction pressure 0.5-5 megapascal, space velocity 400-20000 h⁻¹ (V/V), rotational speed 400-1400 rpm, and H₂/CO = 3-11: 1.

The method can further comprise reducing the catalyst in the presence of pure hydrogen or syngas under the following conditions: reduction temperature 300-350°C, reduction pressure 0.2-1.2 megapascal, rotational speed 400-1400 rpm, space velocity 400-3500 h⁻¹ (V/V), reaction time 6-18 h.

Advantages of the monodisperse iron-based catalyst for Fischer-Tropsch synthesis, preparation method and use thereof as described in the disclosure are summarized as follows.

1. The iron-based catalyst of the disclosure comprises a mesoporous carrier and uniform iron nanoparticles, which makes the reduction degree and carbonization degree of the catalyst easy to control. The iron nanoparticles are encapsulated in the uniform spherical silicon dioxide carrier, which prevents the sintering of the iron nanoparticles and avoids the appearance of difficult reducing components. As a result, the iron-based catalyst has excellent performance and high selectivity of long chain-olefin. The iron nanoparticles are encapsulated in the mesoporous spherical silica and distributed evenly. This structure makes the active sites of the catalyst more consistent, which is conducive to the construction of the active sites. The relatively large mesoporous is conducive to the diffusion of substrates and product molecules, and the size of pore channels can regulate the selectivity of products, thus further improving the selectivity of iron-based catalysts for alpha-olefins.

2. In the preparation of iron-based catalysts, a microemulsion system comprising compressed carbon dioxide in water is used as a template to synthesize the core-shell structure catalysts. Compared with the traditional methods, the morphology of the catalyst is regulated by the pressure of CO₂, and the introduction of CO₂ can also play the role of pore enlargement, thus no need to use the acid, alkali and organic pore enlargers required in the traditional preparation method. By adjusting the pressure of CO₂, the morphology of the catalyst is transformed from a disc-shaped lamination to a uniform spherical shape, the iron nanoparticles are encapsulated in the spherical silicon dioxide, preventing the sintering of iron nanoparticles, and avoiding the appearance of refractory components, thus enhancing the activity of the catalyst and the selectivity of alpha-olefin.

3. The iron-based catalyst can be formed by powder pressing or spray drying, and exhibits good mechanical properties.

4. The monodisperse structure and the metal-carrier interaction of the catalyst facilitate the formation of the active center of the iron-based catalyst suitable for the synthesis of alpha-olefins, so that the catalyst exhibits high selectivity for alpha-olefins. In the presence of the iron-based catalyst, alpha-olefins can be directly synthesized with syngas as raw material.

5. The method uses carbon dioxide as a regulation switch, reduces the use of inorganic mineral acid. The process is environmentally friendly, and develops a new use for carbon dioxide.

### BRIEF DESCRIPTION OF THE DRAWINGS

FIG. 1 is a SEM photo of an iron-based catalyst prepared without or with little CO₂ pressure according to one embodiment of the disclosure;
FIG. 2 is a SEM photo of an iron-based catalyst according to one embodiment of the disclosure; and
FIG. 3 is a TEM photo of an iron-based catalyst according to one embodiment of the disclosure.

### DETAILED DESCRIPTION

To further illustrate, embodiments detailing a monodisperse iron-based catalyst for Fischer-Tropsch synthesis, preparation method and use thereof are described below. It should be noted that the following embodiments are intended to describe and not to limit the disclosure.

### Example 1

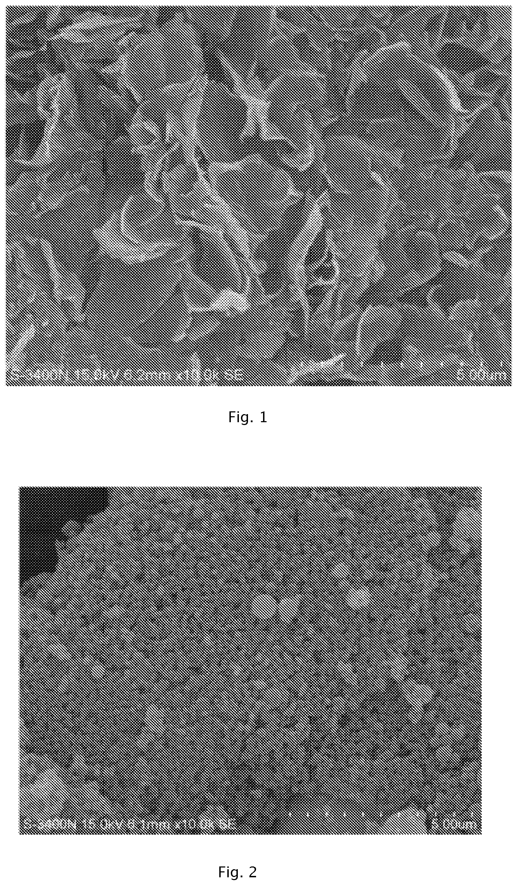

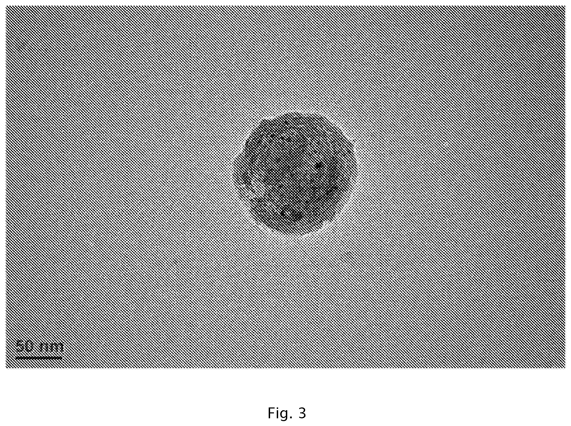

1.22 g of dodecylamine was added to a mixture of 10 mL of ethanol and 90 mL of water and stirred at room temperature for 1 h. Then 0.59 g of iron nanoparticles and 8.16 g of tetraethyl orthosilicate were added. The obtained mixture was transferred to a high-pressure reactor, CO₂ injected, heated to 40°C and stirred for 24 h. The CO₂ pressure was 1.0 megapascal. Thereafter, the high-pressure reactor was cooled and the CO₂ released. The resulting solid was washed with water and suction filtered for several times, dried overnight, to yield a powder. The powder was calcined in a muffle furnace at 500°C for 5 h, tableted, and sieved to yield a monodisperse iron-based catalyst comprising 20 wt. % of iron (20wt%Fe@SiO₂-CO₂-1). With the increase of carbon dioxide, the catalyst is transformed from a disc-shaped lamination (shown in FIG. 1) to homogeneous spherical particles (shown in FIG. 2). The iron nanoparticles are encapsulated in the mesoporous spherical silica and distributed evenly. The particle size of the carrier silica is 150 nm, and the mesoporous diameter of the carrier silica is 2.5 ± 0.4 nm (as shown in FIG. 3).

1.5 mL of 60-80 meshes of the prepared iron-based catalyst was added to a pressurized fixed-bed reactor (*Φ*10×500 mm) and reduced in pure hydrogen by temperature programmed reduction. The reduction conditions were as follows: reduction temperature 400°C, pressure 0.4 megapascal, space velocity 800 h⁻¹ (V/V), and time 12 h. After reduction, the synthesis gas was introduced to the reactor and the reaction conditions were as follows: temperature 260°C, pressure 1.0 megapascal, space velocity 400 h⁻¹ (V/V), H₂/CO = 3/1. The reaction results are shown in Table 2.

15 mL of more than 140 meshes of the prepared iron-based catalyst was added to a 1-L stirred slurry reactor, followed by addition of 500 mL of liquid wax. The resulting mixture was reduced in pure hydrogen by temperature programmed reduction. The reduction conditions were as follows: reduction temperature 400°C, pressure 0.4 megapascal, space velocity 600 h⁻¹ (V/V), rotational speed 600 rpm, and time 12 h. After reduction, the synthesis gas was introduced to the reactor and the reaction conditions were as follows: temperature 260°C, pressure 1.0 megapascal, space velocity 700 h⁻¹ (V/V), rotational speed 600 rpm, and H₂/CO = 3/1. The reaction results are shown in Table 2.

### Example 2

0.5 g of formamide was added to a mixture of 90 mL of ethanol and 10 mL of water and stirred at room temperature for 1 h. Then 0.34 g of iron nanoparticles and 5 g of tetraethyl orthosilicate were added. The obtained mixture was transferred to a high-pressure reactor, CO₂ injected, heated to 45°C and stirred for 22 h. The CO₂ pressure was 4.0 megapascal. Thereafter, the high-pressure reactor was cooled and the CO₂ released. The resulting solid was washed with water and suction filtered for several times, dried overnight, to yield a powder. The powder was calcined in a muffle furnace at 560°C for 4.5 h, tableted, and sieved to yield a monodisperse iron-based catalyst comprising 20 wt. % of iron (20wt%Fe@SiO₂-CO₂-4). The particle size of the carrier silica is 155 nm, and the mesoporous diameter of the carrier silica is 3.7 ± 0.4 nm.

1.5 mL of 60-80 meshes of the prepared iron-based catalyst was added to a pressurized fixed-bed reactor (*Φ*10×500 mm) and reduced in pure hydrogen by temperature programmed reduction. The reduction conditions were as follows: reduction temperature 300°C, pressure 1.2 megapascal, space velocity 3000 h⁻¹ (V/V), and time 12 h. After reduction, the synthesis gas was introduced to the reactor and the reaction conditions were as follows: temperature 260°C, pressure 5.0 megapascal, space velocity 400 h⁻¹ (V/V), H₂/CO = 2/1. The reaction results are shown in Table 2.

15 mL of more than 140 meshes of the prepared iron-based catalyst was added to a 1-L stirred slurry reactor, followed by addition of 500 mL of liquid wax. The resulting mixture was reduced in pure hydrogen by temperature programmed reduction. The reduction conditions were as follows: reduction temperature 300°C, pressure 1 megapascal, space velocity 600 h⁻¹ (V/V), rotational speed 600 rpm, and time 12 h. After reduction, the synthesis gas was introduced to the reactor and the reaction conditions were as follows: temperature 260°C, pressure 1.0 megapascal, space velocity 700 h⁻¹ (V/V), rotational speed 600 rpm, and H₂/CO = 2/1. The reaction results are shown in Table 2.

### Example 3

2 g of piperazine was added to a mixture of 10 mL of ethanol and 90 mL of water and stirred at room temperature for 1 h. Then 1.45 g of iron nanoparticles and 20 g of tetraethyl orthosilicate were added. The obtained mixture was transferred to a high-pressure reactor, CO₂ injected, heated to 35°C and stirred for 26 h. The CO₂ pressure was 6.0 megapascal. Thereafter, the high-pressure reactor was cooled and the CO₂ released. The resulting solid was washed with water and suction filtered for several times, dried overnight, to yield a powder. The powder was calcined in a muffle furnace at 540°C for 5.5 h, tableted, and sieved to yield a monodisperse iron-based catalyst comprising 20 wt. % of iron (20wt%Fe@SiO₂-CO₂-6). The particle size of the carrier silica is 160 nm, and the mesoporous diameter of the carrier silica is 4.6 ± 0.4 nm.

1.5 mL of 60-80 meshes of the prepared iron-based catalyst was added to a pressurized fixed-bed reactor (*Φ*10×500 mm) and reduced in pure hydrogen by temperature programmed reduction. The reduction conditions were as follows: reduction temperature 500°C, pressure 0.2 megapascal, space velocity 800 h⁻¹ (V/V), and time 12 h. After reduction, the synthesis gas was introduced to the reactor and the reaction conditions were as follows: temperature 230°C, pressure 1.0 megapascal, space velocity 2000 h⁻¹ (V/V), H₂/CO = 1/1. The reaction results are shown in Table 2.

15 mL of more than 140 meshes of the prepared iron-based catalyst was added to a 1-L stirred slurry reactor, followed by addition of 500 mL of liquid wax. The resulting mixture was reduced in pure hydrogen by temperature programmed reduction. The reduction conditions were as follows: reduction temperature 400°C, pressure 0.4 megapascal, space velocity 600 h⁻¹ (V/V), rotational speed 600 rpm, and time 12 h. After reduction, the synthesis gas was introduced to the reactor and the reaction conditions were as follows: temperature 240°C, pressure 1.0 megapascal, space velocity 3000 h⁻¹ (V/V), rotational speed 600 rpm, and H₂/CO = 1/1. The reaction results are shown in Table **2**.

### Example 4

1.5 g of aniline was added to a mixture of 10 mL of ethanol and 90 mL of water and stirred at room temperature for 1 h. Then 0.69 g of iron nanoparticles and 10 g of tetraethyl orthosilicate were added. The obtained mixture was transferred to a high-pressure reactor, CO₂ injected, heated to 40°C and stirred for 24 h. The CO₂ pressure was 9.0 megapascal. Thereafter, the high-pressure reactor was cooled and the CO₂ released. The resulting solid was washed with water and suction filtered for several times, dried overnight, to yield a powder. The powder was calcined in a muffle furnace at 500°C for 5 h, tableted, and sieved to yield a monodisperse iron-based catalyst comprising 20 wt. % of iron (20wt%Fe@SiO₂-CO₂-9). The particle size of the carrier silica is 155 nm, and the mesoporous diameter of the carrier silica is 5.3 ± 0.4 nm.

1.5 mL of 60-80 meshes of the prepared iron-based catalyst was added to a pressurized fixed-bed reactor (*Φ*10×500 mm) and reduced in pure hydrogen by temperature programmed reduction. The reduction conditions were as follows: reduction temperature 400°C, pressure 0.4 megapascal, space velocity 800 h⁻¹ (V/V), and time 12 h. After reduction, the synthesis gas was introduced to the reactor and the reaction conditions were as follows: temperature 240°C, pressure 1.0 megapascal, space velocity 800 h⁻¹ (V/V), H₂/CO = 3/1. The reaction results are shown in Table **2**.

15 mL of more than 140 meshes of the prepared iron-based catalyst was added to a 1-L stirred slurry reactor, followed by addition of 500 mL of liquid wax. The resulting mixture was reduced in pure hydrogen by temperature programmed reduction. The reduction conditions were as follows: reduction temperature 400°C, pressure 0.4 megapascal, space velocity 600 h⁻¹ (V/V), rotational speed 600 rpm, and time 12 h. After reduction, the synthesis gas was introduced to the reactor and the reaction conditions were as follows: temperature 260°C, pressure 1.0 megapascal, space velocity 7000 h⁻¹ (V/V), rotational speed 600 rpm, and H₂/CO = 3/1. The reaction results are shown in Table **2**.

### Example 5

0.61 g of laurylamine was added to a mixture of 10 mL of ethanol and 90 mL of water and stirred at room temperature for 1 h. Then 0.59 g of iron nanoparticles and 8.16 g of tetraethyl orthosilicate were added. The obtained mixture was transferred to a high-pressure reactor, CO₂ injected, heated to 40°C and stirred for 24 h. The CO₂ pressure was 4.0 megapascal. Thereafter, the high-pressure reactor was cooled and the CO₂ released. The resulting solid was washed with water and suction filtered for several times, dried overnight, to yield a powder. The powder was calcined in a muffle furnace at 500°C for 5 h, tableted, and sieved to yield a monodisperse iron-based catalyst comprising 20 wt. % of iron (20wt%Fe@SiO₂-CO₂-4). The particle size of the carrier silica is 145 nm, and the mesoporous diameter of the carrier silica is 3.7 ± 0.4 nm.

1.5 mL of 60-80 meshes of the prepared iron-based catalyst was added to a pressurized fixed-bed reactor (*Φ*10×500 mm) and reduced in pure hydrogen by temperature programmed reduction. The reduction conditions were as follows: reduction temperature 400°C, pressure 0.4 megapascal, space velocity 800 h⁻¹ (V/V), and time 12 h. After reduction, the synthesis gas was introduced to the reactor and the reaction conditions were as follows: temperature 260°C, pressure 1.0 megapascal, space velocity 12000 h⁻¹ (V/V), H₂/CO = 3/1. The reaction results are shown in Table **2**.

15 mL of more than 140 meshes of the prepared iron-based catalyst was added to a 1-L stirred slurry reactor, followed by addition of 500 mL of liquid wax. The resulting mixture was reduced in pure hydrogen by temperature programmed reduction. The reduction conditions were as follows: reduction temperature 400°C, pressure 0.4 megapascal, space velocity 600 h⁻¹ (V/V), rotational speed 600 rpm, and time 12 h. After reduction, the synthesis gas was introduced to the reactor and the reaction conditions were as follows: temperature 230°C, pressure 1.0 megapascal, space velocity 1000 h⁻¹ (V/V), rotational speed 600 rpm, and H₂/CO = 3/1. The reaction results are shown in Table **2**.

### Examples 6-10

Examples **6-10** are basically the same as that in Example **5** except the iron content of the iron-based catalyst, the CO₂ pressure, the reaction temperature, and the space velocity. The iron content of the iron-based catalyst and the CO₂ pressure are listed in Table **1**. The reaction temperature, the space velocity, and the reaction results are shown in Table **2**.

The iron content of the iron-based catalyst and the CO₂ pressure in the synthesis process in Examples **1-10** are listed in Table **1**. In addition to the listed component, the rest is silica.

**Table 1 Iron content of iron-based catalysts and CO₂ pressure in the synthesis process in Examples 1-10**

| Example | Fe content (wt. %) | CO₂ pressure (MPa) |
|---|---|---|
| Example 1 | 20% | 1 |
| Example 2 | 20% | 4 |
| Example 3 | 20% | 6 |
| Example 4 | 20% | 9 |
| Example 5 | 20% | 4 |
| Example 6 | 10% | 4 |
| Example 7 | 10% | 1 |
| Example 8 | 30% | 4 |
| Example 9 | 30% | 1 |
| Example 10 | 40% | 4 |

**Table 2 Reactivity of iron-based catalysts of Examples 1-10 in olefin synthesis reaction**

| Iron-based catalysts | Reaction conditions | CO Conversion % | C₁ Selectivity % | C₅+ Selectivity % | Olefin content % |
|---|---|---|---|---|---|
| Example 1 | 260°C, 400 h⁻¹ fixed-bed reactor | 33.4 | 15.7 | 47.8 | 46.1 |
| | 260°C, 700 h⁻¹ slurry reactor | 38.4 | 15.8 | 46.5 | 35.9 |
| Example 2 | 260°C, 400 h⁻¹ fixed-bed reactor | 51.2 | 4.8 | 78.0 | 64.4 |
| | 260°C, 700 h⁻¹ slurry reactor | 40.8 | 5.2 | 77.6 | 65.1 |
| Example 3 | 230°C, 2000 h⁻¹ fixed-bed reactor | 41.3 | 6.1 | 75.2 | 61.6 |
| | 240°C, 3000 h⁻¹ slurry reactor | 32.9 | 5.8 | 76.2 | 63.5 |
| Example 4 | 240°C, 800 h⁻¹ fixed-bed reactor | 65.9 | 9.5 | 73.8 | 57.2 |
| | 260°C, 7000 h⁻¹ slurry reactor | 19.0 | 9.7 | 75.9 | 72.0 |
| Example 5 | 260°C, 12000 h⁻¹ fixed-bed reactor | 26.1 | 4.9 | 78.5 | 62.4 |
| | 230°C, 1000 h⁻¹ slurry reactor | 51.7 | 4.7 | 77.3 | 61.1 |
| Example 6 | 240°C, 6000 h⁻¹ fixed-bed reactor | 28.1 | 7.6 | 75.2 | 63.9 |
| | 250°C, 3000 h⁻¹ slurry reactor | 42.6 | 7.1 | 79.4 | 63.2 |
| Example 7 | 240°C, 6000 h⁻¹ fixed-bed reactor | 28.0 | 5.7 | 80.0 | 61.4 |
| | 250°C, 3000 h⁻¹ slurry reactor | 42.8 | 4.5 | 81.7 | 63.1 |
| Example 8 | 240°C, 2000 h⁻¹ fixed-bed reactor | 51.2 | 11.3 | 73.6 | 62.7 |
| Example 9 | 330°C, 4000 h⁻¹ slurry reactor | 30.3 | 6.8 | 51.9 | 70.3 |
| Example 10 | 270°C, 4000 h⁻¹ slurry reactor | 42.1 | 6.9 | 78.3 | 63.6 |

It will be obvious to those skilled in the art that changes and modifications may be made, and therefore, the aim in the appended claims is to cover all such changes and modifications.

## Claims

1. A monodisperse iron-based catalyst for Fischer-Tropsch synthesis, the catalyst comprising silica and iron; wherein the silica is in the form of a mesoporous spherical particle; the iron is in the form of nanoparticles evenly distributed and encapsulated in the silica; a particle size of the silica is between 140 and 160 nm, and a mesopore of the silica is between 2 and 9 nm.

2. The catalyst of claim 1, wherein the iron accounts for 5-40 wt. % of the catalyst, and the balance is the silica.

3. The catalyst of claim 1 or 2, wherein the particle size of the silica is between 150 and 160 nm, and a mesopore of the silica is between 2.1 and 5.7 nm.

4. The catalyst of claim 1 or 2, wherein the particle size of the silica is between 150 and 155 nm, and a mesopore of the silica is between 3.3 and 4.1 nm.

5. A method of preparing the catalyst of claim 1, the method comprising:
1) mixing ethanol and water with a volume ratio of 1-10: 1-10 to yield an ethanol water, adding 0.005-0.02 g/mL of an organic amine to the ethanol water, to yield a mixture;
2) adding iron nanoparticles to the mixture obtained in 1), followed by addition of 0.05-0.2 g/mL of tetraethyl orthosilicate, to yield a product;
3) introducing 1-15 megapascal CO₂ to the product obtained in 2), heating to 35-45°C for reaction, cooling, and releasing the CO₂, to yield a solid product; and
4) washing the solid product obtained in 3) with water, drying, calcining, to yield the catalyst.

6. The method of claim 5, wherein 3) is performed in a reactor filled with 6-9 megapascal of CO₂ at a temperature of 40-45°C for 22-26 hours.

7. The method of claim 5 or 6, wherein in 4), a calcining temperature is 500-560°C, and a calcining time is 4.5-5.5 hours.

8. The method of claim 5 or 6, wherein in 2), the iron nanoparticles account for 5-40 wt. % of the mixture.

9. A method of preparing alpha-olefin through Fischer-Tropsch synthesis, the method comprising employing syngas as a material and using the catalyst of claim 1, reaction conditions of the Fischer-Tropsch synthesis being as follows: reaction temperature 190-360°C, reaction pressure 0.5-5 megapascal, space velocity 400-20000 h⁻¹ (V/V), rotational speed 400-1400 rpm, and H₂/CO = 3-11: 1.

10. The method of claim 9, further comprising reducing the catalyst in the presence of pure hydrogen or syngas under the following conditions: reduction temperature 300-500°C, reduction pressure 0.2-1.2 megapascal, rotational speed 400-1400 rpm, space velocity 400-3500 h⁻¹ (V/V), and reaction time 6-18 h.
